(19) Europäisches Patentamt
European Patent Office
Office européen des brevets

(11) **EP 3 093 032 B1**

(12) **EUROPÄISCHE PATENTSCHRIFT**

(45) Veröffentlichungstag und Bekanntmachung des Hinweises auf die Patenterteilung:
**19.09.2018 Patentblatt 2018/38**

(51) Int Cl.:
***A61M 1/02*** *(2006.01)*

(21) Anmeldenummer: **15167091.6**

(22) Anmeldetag: **11.05.2015**

(54) **SYSTEM UND VERFAHREN ZUR GEWINNUNG VON SERUM**

SYSTEM AND METHOD FOR OBTAINING SERUM

DISPOSITIF ET PROCÉDÉ DESTINÉS À LA PRODUCTION DE SÉRUM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.11.2016 Patentblatt 2016/46**

(73) Patentinhaber: **3M Innovative Properties Company**
**St. Paul, MN 55133-3427 (US)**

(72) Erfinder: **Bonn, Florian**
**51379 Leverkusen (DE)**

(74) Vertreter: **Hettstedt, Stephan et al**
**3M Deutschland GmbH**
**3M Office of Intellectual Property Counsel**
**Carl-Schurz Str. 1**
**41453 Neuss (DE)**

(56) Entgegenhaltungen:
WO-A1-2014/207140   DE-A1-102005 035 528
DE-A1-102009 022 793   DE-A1-102010 030 238

• **T. BRUNE ET AL: "Quality, Stability, and Safety Data of Packed Red Cells and Plasma Processed by Gravity Separation Using a New Fully Integrated Hollow-Fibre Filter Device", ADVANCES IN HEMATOLOGY, Bd. 45, Nr. 3, 1. Januar 2009 (2009-01-01) , Seiten 287-6, XP055223360, ISSN: 1687-9104, DOI: 10.1111/j.1423-0410.2005.00660.x**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung sowie ein Verfahren zur Gewinnung von Serum aus Vollblut.

In zunehmenden Maße werden Blutprodukte zur Therapie von Patienten benötigt. So wird Eigen-oder Fremdserum als Blutprodukt bei der Behandlung von Entzündungen eingesetzt. Als Beispiel sei die Behandlung von Augenerkrankungen wie Hornhautproblemen mittels Eigenserumaugentropfen genannt. Auch bei Wundbehandlungen kann Eigen- oder auch Fremdserum der Wunde zugeführt wird, um den Heilungsprozess zu beschleunigen.

Die Herstellung von Serum aus Vollblut erfolgt vielfach mittels Zentrifugen, in denen koaguliertes Blut in seine Bestandteile aufgetrennt wird. Der Zenrifugierprozess kann auch in Kombination mit speziellen Vorrichtungen zum zur Abtrennung und Lagerung von Blutkomponenten betrieben werden, wie sie z.B. aus der EP 2 174 676 A1 bekannt sind. Derartige Prozesse sind jedoch aufwändig in der Durchführung und kostenintensiv. Darüber hinaus ist über bekannte Prozessen hergestelltes Serum oftmals nicht frei von Feststoffbestandteilen, wie beispielsweise zellulären Bestandteilen des Bluts. Schließlich erlauben verschiedene bekannte Verfahren zur Herstellung von Serum nicht eine direkte Portionierung des Serums in Aliquots, die bei verschiedenen Anwendungen auch im Bereich der Therapie von Patienten mit Serum benötigt werden.

Aus der DE 10 2009 022 793 A1 ist eine Vorrichtung zur Entnahme von Blut und Herstellung von Blutprodukten bekannt.

Aus der WO2014/207140 A1 ist ein Vollbluthohlfasermembranfiltermedium bekannt, das eine Porengröße hat, die Durchlässigkeit gegenüber Blutplasma oder -serum gewährleistet, aber Blutzellen zurückhält.

Daher besteht der Wunsch nach einer einfach zu handhabenden Vorrichtung sowie nach einem vereinfachten Verfahren zur Gewinnung von zellfreiem Blutserum.

Die Aufgabe wird durch eine Vorrichtung nach Anspruch 1 zur Gewinnung von zellfreiem und sterilem Serum aus Vollblut gelöst, wobei die Vorrichtung umfasst:

- einen Blutbeutel zur Aufnahme von Vollblut und zur Koagulation des Vollbluts in einen die Zellbestandteile des Bluts enthaltenden Feststoffanteil und in eine Serum enthaltene Flüssigkeit, wobei der Blutbeutel einen Auslass zur Ableitung der Flüssigkeit aus dem Blutbeutel aufweist,
- einen mit dem Blutbeutel über den Auslass des Blutbeutels in Fluidverbindung stehenden Filtermodul,
- wobei der Blutbeutel im Bereich des Auslasses eine Sperre aufweist, welche zum Rückhalt zumindest eines Großteils des Feststoffanteils und zum Durchtritt der Flüssigkeit geeignet ist,
  der Filtermodul ein Gehäuse mit einem Innenraum und einer den Innenraum begrenzenden Innenwand aufweist, in dem eine zum Rückhalt von in der Flüssigkeit enthaltenen Feststoffanteilen geeigneten semipermeable Membran angeordnet ist, die den Innenraum in einen Retentatraum und einen Permeatraum unterteilt,
- wobei die semipermeable Membran des Filtermoduls ein Bündel von Hohlfasermembranen ist und der Retentatraum durch die Lumina der Hohlfasermembranen und der Permeatraum durch den die Hohlfasermembranen umgebenden und durch die Innenwand des Gehäuses begrenzten Außenraum ausgebildet wird,
- wobei die semipermeable Membran eine Auftrennung der aus dem Blutbeutel abgeleiteten Flüssigkeit in Serum als Permeat und in ein Retentat, in dem eventuell in der Flüssigkeit enthaltene partikulärer Bestandteile verbleiben, ermöglicht und einen Transmembranfluss im Bereich von 15.000 bis 30.000 l/ (m$^2$*h*bar) sowie eine nominelle Pore von 0,2 $\mu$m aufweist und
- wobei der Filtermodul eine Einlasseinrichtung zum Einleiten der aus dem Blutbeutel abgeleiteten Flüssigkeit in den Retentatraum und einen Permeatauslass zum Ableiten des Serums aus dem Permeatraum aufweist, sowie
- mindestens einen Aufnahmebehälter für Serum, welcher eine Einlassöffnung aufweist, wobei der Permeatauslass des Filtermoduls und die Einlassöffnung des mindestens einen Aufnahmebehälters über eine Verbindungsleitung verbunden sind.

Die Erfindung betrifft des Weiteren ein Verfahren nach Anspruch 6 zur Herstellung eines zellfreien und sterilen Serums aus Vollblut, umfassend die Schritte

- Vorlegen von Vollblut in einem Blutbeutel,
- Koagulation des Vollbluts im Blutbeutel in einen die Zellbestandteile des Bluts enthaltenden Feststoffanteil und in eine Serum enthaltene Flüssigkeit,
- Ausleiten der das Serum enthaltenden Flüssigkeit über einen Auslass des Blutbeutels, wobei der Blutbeutel im Bereich des Auslasses eine Sperre aufweist, welche zum Rückhalt zumindest eines Großteils des Feststoffanteils geeignet ist,
- Einleiten der Flüssigkeit über eine Einlasseinrichtung zum Einleiten der Flüssigkeit in einen Retentatraum eines Filtermoduls,
- Filtern der Flüssigkeit mittels einer im Filtermodul angeordneten und zum Rückhalt von in der Flüssigkeit enthaltenen Feststoffanteilen geeigneten semipermeablen Membran mit einem Transmembranfluss im Bereich von 15.000 bis 30.000 l/(m$^2$*h*bar) sowie einer nominellen Pore von 0,2 µm, wodurch zellfreies Serum als Permeat und ein Retentat erhalten wird, in dem Feststoffanteile der Flüssigkeit verbleiben,

- Ausleiten des zellfreien Serums über einen Permeatauslass des Filtermoduls und
- Einleiten des zellfreien Serums in mindestens einen Aufnahmebehälter über eine Einlassöffnung des mindestens einen Aufnahmebehälters, wobei der Permeatauslass und die Einlassöffnung über eine Verbindungsleitung verbunden sind.

Der im vorliegenden Verfahren eingesetzte Filtermodul weist ein Gehäuse mit einem Innenraum und einer den Innenraum begrenzenden Innenwand auf, in dem die semipermeable Membran angeordnet ist. Die semipermeable Membran unterteilt den Innenraum des Filtermoduls in einen Retentatraum und einen Permeatraum. Aus dem Blutbeutel abgeleitete Flüssigkeit wird über die Einlasseinrichtung zum Einleiten der Flüssigkeit in den Filtermodul und in den Retentatraum eingeleitet. Aus dem Permeatraum strömt die Flüssigkeit in die semipermeable Membran ein und wird dort gefiltert und von in der Flüssigkeit enthaltenen Feststoffanteilen befreit. Als Filtrat, d.h. als Permeat wird dann zellfreies Serum erhalten. Restliche Flüssigkeit verbleibt zusammen mit dem Feststoffanteil als Retentat im Retentatraum. Für die Durchführung des vorliegenden Verfahrens ist die zuvor beschriebene Vorrichtung zur Gewinnung von Serum aus Vollblut bestens geeignet.

Die vorliegende Vorrichtung bzw. das vorliegende Verfahren erlaubt in besonders wirtschaftlicher Weise eine sichere Gewinnung eines zellfreien Blutserums. Die Abtrennung des zellfreien Serums kann allein unter dem Einfluss der Schwerkraft erfolgen, wobei kein Bedarf an Elektrizität und elektronischen Geräten und auch kein Bedarf an speziell ausgebildetem Personal besteht. Hierzu werden in der Anwendung bzw. beim vorliegenden Verfahren Blutbeutel, Filtermodul und der mindestens eine Aufnahmebehälter über eine geeignete Haltevorrichtung vorzugsweise in definierten senkrechten Abständen zueinander angeordnet, so dass das Ausfließen aus dem Blutbeutel in den Filtermodul und im weiteren aus dem Filtermodul in den mindestens einen Aufnahmebehälter allein unter dem Einfluss der Schwerkraft erfolgt, ohne dass beipielsweise Pumpen u.ä erforderlich sind.

Bei der vorliegenden Vorrichtung zur Gewinnung von Serum bzw. beim vorliegenden Verfahren wird in der Anwendung der Blutbeutel mit einer vorgegebenen Menge an Vollblut gefüllt. Das im Blutbeutel befindliche Blut wird dann einer Koagulation oder Blutgerinnung unterworfen. Die Koagulation ist ein komplexer Vorgang, der wie eine Kettenreaktion unter Beteiligung verschiedenen Gerinnungsfaktoren (wie Thrombozyten, Fibrinogen, Calcium und Vitamin K) abläuft. Die Koagulation des Blutes wird durch die Entnahme des Blutes und das Fehlen von Antikoagulationsmitteln wie Citrat oder Heparin im Blutbeutel ausgelöst.

Infolge der Blutgerinnung trennt sich das Blut in einen Feststoffanteil (Koagulum), der die zellulären Bestandteile wie z.B. rote Blutkörperchen, Blutplättchen und weiße Blutkörperchen enthält, und eine flüssige Phase, die bis auf die bei der Gerinnung verbrauchten Gerinnungsfaktoren alle natürlicherweise in der Blutflüssigkeit gelösten Stoffe enthält. Dabei liegt der Feststoffanteil als gallertartige Masse vor.

Der Blutbeutel weist zur Aufnahme des zu koagulierenden Vollbluts einen Einlass auf. Der Einlass kann auch mit z.B. über eine geeignete Schlauchverbindung mit einem Element verbunden sein, das mit einem oder mehreren Blutgefäßen eines Spenders koppelbar ist. Bei diesem Element kann es sich um eine Spenderkanüle herkömmlicher Art handeln. Des Weiteren kann mit der Schlauchverbindung zwischen z.B. der Spenderkanüle und dem Einlass des Blutbeutels ein Vorspenderbeutel für Testwecke über einen Abzweig verbunden sein, der eine Untersuchung des Spenderbluts aus dem Blutbeutel auf seine Bauchbarkeit ermöglicht.

Der von der Vorrichtung zur Gewinnung von Serum umfasste bzw. im vorliegenden Verfahren eingesetzte Blutbeutel weist im Bereich seines Auslasses eine Sperre auf, durch die zumindest ein Großteil des Feststoffanteils, d.h. des in Gestalt einer gallertartigen Masse vorliegenden Koagulums, zurückgehalten werden kann und die den Durchtritt des Flüssigkeitsanteils erlaubt. Hierzu kann die Sperre eine Verengung des Strömungsquerschnitts im Bereich des Auslasses sein, die zumindest im wesentlichen nur den Durchtritt des Flüssigkeitsanteils ermöglicht. Die Sperre kann auch eine Klemme sein, die im Bereich des Auslasses einen genügend großen Spalt bzw. Strömungsquerschnitt für den Durchtritt der Flüssigkeit aus dem Blutbeutel aufweist. Die Sperre kann auch ein von außen einstellbares Stellglied sein, das eine Einstellung der Querschnittverengung von außen ermöglicht. Hierdurch wird ermöglicht, dass der Strömungsquerschnitt zunächst geschlossen durch Sperre zunächst geschlossen ist, bis die Koagulation des Bluts erfolgt ist. Nach erfolgter Koagulation kann dann die Sperre soweit geöffnet werden, dass die Flüssigkeit hindurchtritt, das Koagulum jedoch nicht. Die Sperre kann im Auslass der Blutbeutels angebracht sein, sie kann sich jedoch auch in dem Verbindungsschlauch oder der Verbindungsleitung angeordnet sein, der bzw. die die Fluidverbindung zwischen Blutbeutel und Filtermodul herstellt.

Der Auslass des Blutbeutels steht mit dem Filtermodul in Fluidverbindung, beispielsweise über einen geeigneten Schlauchabschnitt. Die Verbindung zwischen Blutbeutel und Filtermodul ist vorzugsweise lösbar ausgeführt, beispielsweise durch geeignete Male-/Female-Konnektoren etwa in Gestalt von Luer-Lock-Konnektoren. Alternativ kann die Verbindung zwischen Blutbeutel und Filtermodul, d.h. letztlich zwischen Blutbeutel und Schlauchabschnitt, zwischen Schlauchabschnitt und Filtermodul oder zwischen Teilabschnitten des Schlauchabschnitts auch als sterile Schweißverbindung ausgeführt sein, wie sie mittels eines Sterilkonnektors (z.B. TSCD® II Sterile Tubing Welder, Fa. Terumo) hergestellt werden kann.

Die im Filtermodul bzw. im Innenraum des Gehäuses des Filtermoduls angeordnete und zum Rückhalt von in der Flüssigkeit enthaltenen Feststoffanteilen geeignete semipermeable Membran kann in Gestalt einer Flachmembran oder einer Hohlfasermembran ausgeführt sein. Bevorzugt ist die semipermeable Membran ein Bündel von Hohlfasermembranen. In diesem Fall wird der Retentatraum durch die Lumina der Hohlfasermembranen und der Permeatraum durch den die Hohlfasermembranen umgebenden und durch die Innenwand des Gehäuses begrenzten Außenraum ausgebildet. Dabei ist in einer besonders bevorzugten Ausführungsform das im Filtermodul angeordnete Bündel von Hohlfasermembranen U-förmig ausgebildet. In diesem Fall hat das Gehäuse des Filtermoduls einen Deckel und einen Boden und die Einlasseinrichtung zum Einleiten der aus dem Blutbeutel abgeleiteten Flüssigkeit in den Retentatraum sowie der Retentatauslass zum Ableiten von Retentat aus dem Retentatraum sind deckelseitig angeordnet. Der Permeatauslass zum Ableiten des Permeats, d.h. des Serum, aus dem Permeatraum ist bei diesem Filtermodul bodenseitig angeordnet.

In einer Ausführungsform kann der Filtermodul neben der Einlasseinrichtung zum Einleiten der aus dem Blutbeutel abgeleiteten Flüssigkeit in den Retentatraum und dem Permeatauslass zum Ableiten des Serums aus dem Permeatraum des Weiteren einen Retentatauslass zum Ableiten von Retentat aus dem Filtermodul umfassen. Dies erlaubt eine Fahrweise des Filtermoduls, bei der ein Teil der Flüssigkeit mit darin enthaltenem Feststoff aus dem Retentatraum abgeführt werden kann. In diesem Fall steht der Retentatauslass dann mit einem Auffangbehälter für Retentat z.B. über einen Verbindungsschlauch in Fluidverbindung.

Um einen genügend hohen Permeatfluss durch die semipermeable Membran des Filtermoduls insbesondere bei der Gewinnung von Serum unter dem Einfluss der Schwerkraft generieren zu können, weist die semipermeable Membran des Filtermoduls einen Transmembranfluss im Bereich von 15.000 bis 30.000 l/(m$^2$·h·bar) auf.

Mittels der im semipermeablen Membran des Filtermoduls werden zelluläre Bestandteile aus der dem Filtermodul aus dem Blutbeutel zugeführten, das Serum enthaltenden Flüssigkeit entfernt. Hierzu weist die semipermeable Membran des Filtermoduls in einer bevorzugten Ausführungsform einen nach der Blaspunktmethode ermittelten maximalen Trennporendurchmesser $d_{max}$ im Bereich von 0,3 bis 0,7 $\mu$m auf. Hierdurch lässt sich erreichen, dass die Membran auf der einen Seite einen genügenden Rückhalt gegenüber zellulären Bestandteilen in der ihr zugeführten Flüssigkeit aufweist. Auf der anderen Seite lassen sich so genügend hohe Transmembranflüsse realisieren, die einen Betrieb der Vorrichtung bzw. eine Fahrweise des Verfahrens allein unter dem Einfluss der Gravitation ermöglicht. Besonders bevorzugt weist die Membran einen nach der Blaspunktmethode ermittelten maximalen Trennporendurchmesser $d_{max}$ im Bereich von 0,4 bis 0,6 $\mu$m auf. Der Durchmesser der maximalen Trennpore $d_{max}$ lässt sich mittels der Blaspunktmethode (ASTM Nr. 128-99 und F 316-03) bestimmen, wozu beispielsweise die in der DE-A-36 17 724 beschriebene Methode geeignet ist. Dabei ergibt sich $d_{max}$ aus dem zum Blaspunkt zugehörigen Gasraumdruck $P_B$ nach der Beziehung $d_{max} = \sigma_B / P_B$. Hierbei ist $\sigma_B$ eine Konstante, die hauptsächlich von der bei der Messung eingesetzten benetzenden Flüssigkeit abhängig ist. Für IPA beträgt $\sigma_B$ bei 25°C 0,61 $\mu$m·bar.

Erfindungsgemäß weist die semipermeable Membran des Filtermoduls eine nominelle Pore von 0,2 $\mu$m auf. Bei Einsatz einer solchen Membran lässt sich mittels der vorliegenden Vorrichtung bzw. mittels des vorliegenden Verfahrens ein steriles Serum herstellen. Dabei wird die nominelle Pore über das Rückhaltevermögen der Membran gegenüber spezifischen Mikroorganismen definiert. Membranen mit einer nominellen Pore von 0,2 $\mu$m werden generell als bakteriendicht bezeichnet, da sie Bakterien der Gattung Brevundimonas diminuta zurückhalten. Ebenso halten sie natürlich z.B. auch Bakterien der Gattung Serratia marcescens zurück, für die eine Membran mit nomineller Pore von 0,45 $\mu$m ausreichend wäre. Die Prüfungen bzw. die Ermittlung der nominellen Porengrößen sind beispielsweise in der HIMA-Vorschrift No. 3, Vol. 4, 1982 (Health Industry Manufacturers Association) beschrieben.

Die vorliegende Vorrichtung zur Gewinnung von zellfreiem Serum aus Vollblut umfasst mindestens einen Aufnahmebehälter für Serum, welcher eine Einlassöffnung aufweist, wobei der Permeatauslass des Filtermoduls und die Einlassöffnung des mindestens einen Aufnahmebehälters über eine Verbindungsleitung verbunden sind. Ebenso wird im vorliegenden Verfahren ein solcher Aufnahmebehälter eingesetzt. Bei dem mindestens einen Aufnahmebehälter kann es sich beispielsweise um einen Sammelbeutel aus einem elastischen Kunststoffmaterial handeln.

In einer bevorzugten Ausführungsform handelt es sich bei dem mindestens einen Aufnahmebehälter für Serum um mindestens zwei Aufnahmebehälter, die zueinander parallel angeordnet sind, wobei die Einlassöffnungen der Aufnahmebehälter über jeweils eine Verbindungsleitung mit dem Permeatauslass des Filtermoduls verbunden sind. Die Verbindungsleitungen sind dabei zweckmäßigerweise über einen oder mehrere Verteiler z.B. in Gestalt eines oder mehrerer Y-Verbinder oder eines Mehrwegehahns mit dem Permeatauslass verbunden.

In einer weiteren bevorzugten Ausführungsform sind mindestens zwei Aufnahmebehälter für Serum zueinander in Reihe angeordnet, wobei die Aufnahmebehälter miteinander in Fluidverbindung stehen und wobei die Einlassöffnung des ersten Aufnahmebehälters über eine Verbindungsleitung mit dem Permeatauslass des Filtermoduls verbunden ist.

Derartige Anordnungen von zueinander parallel oder in Reihe angeordneten Aufnahmebehältern sind insbesondere dann von Vorteil, wenn das im Filtermodul erhaltene zellfreie Serum direkt in Teilmengen, d.h. in Aliquots unterteilt werden soll. Hierbei können die Aufnahmebehälter auch so ausgeführt sein, dass sie zur direkten Verabreichung des darin enthaltenen Serums geeignet sind, z.B. zur Verabreichung als Eigenserumaugentropfen. Hierfür geeignete und zunächst in Serie geschaltete Behälter zur Verabreichung werden beispielsweise in der WO 2010/136535 beschrieben.

Die Verbindung des Permeatauslasses mit dem mindestens einen Aufnahmebehälter kann vorzugsweise lösbar ausgeführt sein, beispielsweise in Gestalt geeigneter Male-/Female-Konnektoren, wie z.B. von Luer-Lock-Konnektoren. Alternativ kann die Verbindung zwischen Permeatauslass und dem mindestens einen Aufnahmebehälter zwei Schlauchabschnitte umfassen, die über eine sterile Schweißverbindung verbunden sind oder verbindbar sind, wie sie mittels eines Sterilkonnektors (z.B. TSCD® II Sterile Tubing Welder, Fa. Terumo) hergestellt werden kann.

Insgesamt kann mittels derartiger steriler oder zumindest weitgehend steriler Verbindungen zwischen den Elementen der Vorrichtung eine Höchstmaß an Sicherheit hinsichtlich einer Keimfreiheit des erhaltenen zellfreien Serums erreicht werden.

Für die Charakterisierung der Eigenschaften der im Wundversorgungssystem eingesetzten Kapillarmembranen bzw. Flachmembranen werden die folgenden Messmethoden zu Grunde gelegt:

Transmembranfluss (Wasserpermeabilität) für Kapillarmembranen:

Aus den zu prüfenden Kapillarmembranen wird eine Prüfzelle mit definierter Kapillarmembranzahl und Länge gefertigt. Die Kapillarmembranen werden dafür beidseitig an ihren Enden in ein Polyurethanharz eingebettet. Nach dem Aushärten des Harzes werden die Einbettungen auf eine Länge von ca. 30 mm geschnitten, wobei die Lumina der Kapillarmembranen durch den Schnitt geöffnet werden. Die Kapillarlumina in den Einbettungen müssen auf Durchgängigkeit überprüft werden. Die freie Länge der Kapillarmembranen zwischen den Einbettungen beträgt üblicherweise 120 +/- 10 mm. Die Anzahl der Kapillarmembranen ist so zu bemessen, dass unter Berücksichtigung der freien Länge und des Innendurchmessers der Kapillarmembranen eine Filtrationsfläche von ca. 30 cm$^2$ in der Prüfzelle bereitgestellt wird.

Die Prüfzelle wird mit in eine Prüfapparatur eingebunden und mit auf 25°C temperiertem ultrafiltriertem und vollentsalztem Wasser bei einem definiertem Prüfdruck (ca. 0,4 bar) durchströmt. Die während einer Messzeit von 2 min erhaltene filtrierte Wassermenge, d.h. das während der Messung erzeugte Permeat wird gravimetrisch oder volumetrisch erfasst. Vor Beginn der Messung muss die Anlage luftfrei gespült werden. Zur Bestimmung des TMF werden in der Prüfapparatur der Eingangs- und Ausgangsdruck an der Prüfzelle gemessen. Die Messung wird bei 25°C durchgeführt.

Der Transmembranfluss TMF wird nach der Formel (I)

$$TMF = \frac{V_W}{\Delta t \cdot A_M \cdot \Delta p} \quad \left[\frac{ml}{cm^2 \cdot min \cdot bar}\right] \qquad (I)$$

ermittelt. Hierbei sind:

$V_W$  = durch die Membranprobe während der Messzeit hindurch geströmte Wasservolumen [ml]
$\Delta t$  = Messzeit [min]
$A_M$  = durchströmte Fläche der Membranprobe (üblicherweise 30 cm$^2$)
$\Delta p$  = eingestellter Druck während der Messung [bar]

**Patentansprüche**

1. Vorrichtung zur Gewinnung von zellfreiem und sterilem Serum aus Vollblut, wobei die Vorrichtung umfasst:

- einen Blutbeutel zur Aufnahme von Vollblut und zur Koagulation des Vollbluts in einen die Zellbestandteile des Bluts enthaltenden Feststoffanteil und in eine Serum enthaltene Flüssigkeit, wobei der Blutbeutel einen Auslass zur Ableitung der Flüssigkeit aus dem Blutbeutel aufweist,
- einen mit dem Blutbeutel über den Auslass des Blutbeutels in Fluidverbindung stehenden Filtermodul, wobei
- der Blutbeutel im Bereich des Auslasses eine Sperre aufweist, welche zum Rückhalt zumindest eines Großteils des Feststoffanteils und zum Durchtritt der Flüssigkeit geeignet ist,
- der Filtermodul ein Gehäuse mit einem Innenraum und einer den Innenraum begrenzenden Innenwand aufweist, in dem eine zum Rückhalt von in der Flüssigkeit enthaltenen Feststoffanteilen geeigneten semipermeable Membran angeordnet ist, die den Innenraum in einen Retentatraum und einen Permeatraum unterteilt,

- wobei die semipermeable Membran des Filtermoduls ein Bündel von Hohlfasermembranen ist und der Retentatraum durch die Lumina der Hohlfasermembranen und der Permeatraum durch den die Hohlfasermembranen umgebenden und durch die Innenwand des Gehäuses begrenzten Außenraum ausgebildet wird,

- wobei die semipermeable Membran eine Auftrennung der aus dem Blutbeutel abgeleiteten Flüssigkeit in Serum als Permeat und in ein Retentat, in dem eventuell in der Flüssigkeit enthaltene partikulärer Bestandteile verbleiben, ermöglicht und einen Transmembranfluss im Bereich von 15.000 bis 30.000 l/(m$^2$·h·bar) sowie eine nominelle Pore von 0,2 $\mu$m aufweist und

- wobei der Filtermodul eine Einlasseinrichtung zum Einleiten der aus dem Blutbeutel abgeleiteten Flüssigkeit in den Retentatraum und einen Permeatauslass zum Ableiten des Serums aus dem Permeatraum aufweist, sowie

- mindestens einen Aufnahmebehälter für Serum, welcher eine Einlassöffnung aufweist, wobei der Permeatauslass des Filtermoduls und die Einlassöffnung des mindestens einen Aufnahmebehälters über eine Verbindungsleitung verbunden sind.

2. Vorrichtung nach Anspruch 1, **dadurch gekennzeichnet, dass** das Bündel von Hohlfasermembranen U-förmig ausgebildet ist.

3. Vorrichtung nach Anspruch 1 oder 2, **dadurch gekennzeichnet, dass** der Filtermodul des Weiteren einen Retentatauslass zum Ableiten des Retentats aufweist.

4. Vorrichtung nach einem oder mehreren der Ansprüche 1 bis 3, **dadurch gekennzeichnet, dass** sie mindestens zwei Aufnahmebehälter für Serum umfasst, die zueinander parallel angeordnet sind, wobei die Einlassöffnungen der Aufnahmebehälter über jeweils eine Verbindungsleitung mit dem Permeatauslass des Filtermoduls verbunden sind.

5. Vorrichtung nach einem oder mehreren der Ansprüche 1 bis 4, **dadurch gekennzeichnet, dass** sie mindestens zwei Aufnahmebehälter für Serum umfasst, die zueinander in Reihe angeordnet sind, wobei die Aufnahmebehälter miteinander in Fluidverbindung stehen und wobei die Einlassöffnung des ersten Aufnahmebehälters über eine Verbindungsleitung mit dem Permeatauslass des Filtermoduls verbunden ist.

6. Verfahren zur Gewinnung von zellfreiem und sterilem Serum aus Vollblut umfassend die Schritte

- Vorlegen von Vollblut in einem Blutbeutel,

- Koagulation des Vollbluts im Blutbeutel in einen die Zellbestandteile des Bluts enthaltenden Feststoffanteil und in eine Serum enthaltene Flüssigkeit,

- Ausleiten der das Serum enthaltenden Flüssigkeit über einen Auslass des Blutbeutels, wobei der Blutbeutel im Bereich des Auslasses eine Sperre aufweist, welche zum Rückhalt zumindest eines Großteils des Feststoffanteils geeignet ist,

- Einleiten der Flüssigkeit über eine Einlasseinrichtung zum Einleiten der Flüssigkeit in einen Retentatraum eines Filtermoduls,

- Filtern der Flüssigkeit mittels einer im Filtermodul angeordneten und zum Rückhalt von in der Flüssigkeit enthaltenen Feststoffanteilen geeigneten semipermeablen Membran mit einem Transmembranfluss im Bereich von 15.000 bis 30.000 l/(m$^2$·h·bar) sowie einer nominellen Pore von 0,2 $\mu$m, wodurch zellfreies und steriles Serum als Permeat und ein Retentat erhalten wird, in dem Feststoffanteile der Flüssigkeit verbleiben,

- Ausleiten des zellfreien Serums über einen Permeatauslass des Filtermoduls und

- Einleiten des zellfreien Serums in mindestens einen Aufnahmebehälter über eine Einlassöffnung des mindestens einen Aufnahmebehälters, wobei der Permeatauslass und die Einlassöffnung über eine Verbindungsleitung verbunden sind.

7. Verfahren zur Gewinnung von zellfreiem Serum aus Vollblut nach Anspruch 6, **dadurch gekennzeichnet, dass** das Ausfließen aus dem Blutbeutel in den Filtermodul und im weiteren aus dem Filtermodul in den mindestens einen Aufnahmebehälter allein unter dem Einfluss der Schwerkraft erfolgt

8. Verfahren zur Gewinnung von zellfreiem Serum aus Vollblut nach Anspruch 6 oder 7, **dadurch gekennzeichnet, dass** eine Vorrichtung nach einem oder mehreren der Ansprüche 1 bis 5 eingesetzt wird.

**Claims**

1. Device for obtaining cell-free and sterile serum made from whole blood, wherein the device comprises:

   - a blood bag for holding whole blood and for coagulating the whole blood into a solid portion containing the cell components of the blood and into a fluid containing serum, wherein the blood bag has an outlet for discharging the fluid from the blood bag;
   - a filter module with a fluid connection to the blood bag by means of the outlet of the blood bag; wherein
   - the blood bag has a barrier in the area of the outlet, which barrier is suitable for retaining at least a large part of the solid portion and for enabling the fluid to flow through;
   - the filter module has a housing with an interior and an inner wall bordering the interior in which a semi-permeable membrane suitable for retaining solid portions contained in the fluid is arranged, which membrane subdivides the interior into a retentate space and a permeate space;
   - wherein the semi-permeable membrane of the filter module is a bundle of hollow-fiber membranes and the retentate space is formed by the lumens of the hollow-fiber membranes and the permeate space is formed by the outer space bordering the inner wall of the housing and surrounding the hollow-fiber membranes;
   - wherein the semi-permeable membrane enables a separation of the fluid discharged from the blood bag into serum as a permeate and into retentate, in which particular components potentially contained in the fluid are retained, and has a transmembrane flow in a range of from 15,000 to 30,000 $L/(m^2 \cdot h \cdot bar)$, as well as a nominal pore size of 0.2 $\mu$m; and
   - wherein the filter module has an inlet device for introducing the fluid discharged from the blood bag into the retentate space and a permeate outlet for discharging the serum from the permeate space; as well as
   - at least one collection container for serum which has an inlet opening, wherein the permeate outlet of the filter module and the inlet opening of the at least one collection container are connected by means of a connection line;

2. The device according to claim 1, **characterized in that** the bundle of hollow-fiber membranes is formed in the shape of a U.

3. The device according to either claim 1 or 2, **characterized in that** the filter module furthermore has a retentate outlet for discharging the retentate.

4. The device according to one or more of claims 1 to 3, **characterized in that** it comprises at least two collection containers for serum which are arranged parallel to one another, wherein the inlet openings of the collection containers are connected to the permeate outlet of the filter module by means of a connection line for each.

5. The device according to one or more of claims 1 to 4, **characterized in that** it comprises at least two collection containers for serum which are arranged in series with one another, wherein the collection containers have a fluid connection and wherein the inlet opening of the first collection container is connected to the permeate outlet of the filter module by means of a connection line.

6. Method for obtaining cell-free and sterile serum made from whole blood, comprising the following steps:

   - placement of whole blood into a blood bag;
   - coagulation of the whole blood in the blood bag into a solid portion containing the cell components of the blood and into a liquid containing serum;
   - removal of the fluid containing the serum by means of an outlet of the blood bag, wherein the blood bag has a barrier in the area of the outlet, which barrier is suitable for retaining at least a large part of the solid portion;
   - introduction of the fluid into a retentate space of a filter module by means of an inlet device for introducing the fluid;
   - filtering of the fluid by means of a semi-permeable membrane, which is arranged in the filter module and is suitable for retaining solid portions contained in the fluid, having a transmembrane flow in a range of from 15,000 to 30,000 $L/(m^2 \cdot h \cdot bar)$, as well as a nominal pore size of 0.2 $\mu$m, whereby cell-free and sterile serum is obtained as a permeate and a retentate, in which the solid portions of the fluid remain;
   - removal of the cell-free serum by means of a permeate outlet of the filter module and
   - introduction of the cell-free serum into at least one collection container by means of an inlet opening of the at least one collection container, wherein the permeate outlet and the inlet opening are connected by means of a connection line;

7. The method for obtaining cell-free serum from whole blood according to claim 6, **characterized in that** the outflow from the blood bag to the filter module and furthermore from the filter module into the at least one collection container takes place solely under the effect of gravity.

8. The method for obtaining cell-free serum from whole blood according to either claim 6 or 7, **characterized in that** a device according to one or more of claims 1 to 5 is used.

**Revendications**

1. Dispositif pour recueillir du sérum acellulaire et stérile à partir de sang total, le dispositif comprenant :

- une poche à sang pour la réception du sang total et la coagulation du sang total en une composante solide contenant les composants cellulaires du sang et un liquide contenant du sérum, dans lequel la poche à sang présente une sortie pour l'évacuation du liquide hors de la poche à sang,
- un module de filtration en communication fluidique avec la poche à sang par le biais de la sortie de la poche à sang, dans lequel
- la poche à sang, dans la zone de la sortie, présente un dispositif d'arrêt, qui est approprié à retenir au moins une grande partie de la composante liquide et à laisser passer le liquide,
- le module de filtration présente un boîtier avec un espace interne et une paroi interne délimitant l'espace interne, dans lequel une membrane semi-perméable est disposée, appropriée à retenir les composantes solides contenues dans le liquide, laquelle membrane sépare l'espace interne en un espace de rétentat et un espace de perméat,
- dans lequel la membrane semi-perméable du module de filtration est un faisceau de membranes de fibres creuses et l'espace de rétentat est formé par les lumières de membranes de fibres creuses et l'espace de perméat est formé par l'espace externe entourant les membranes de fibres creuses et délimité par la paroi interne du boîtier,
- dans lequel la membrane semi-perméable permet une séparation du liquide évacué hors de la poche à sang en sérum en tant que perméat et en un rétentat, dans lequel peuvent subsister éventuellement les composants particulaires contenus dans le liquide et présente un écoulement transmembranaire dans la plage de 150 000 à 300 000 dm$^3$/(m$^2$·h·MPa) (15 000 à 30 000 L/(m$^2$·h·bar)) ainsi qu'un pore nominal de 0,2 $\mu$m et
- dans lequel le module de filtration présente un dispositif d'entrée pour faire pénétrer le liquide évacué hors de la poche à sang dans l'espace de rétentat et une sortie de perméat afin d'évacuer le sérum hors de l'espace de perméat, ainsi que
- au moins un contenant de réception pour le sérum, qui présente une ouverture d'entrée, dans lequel la sortie de perméat du module de filtration et l'ouverture d'entrée dudit au moins un contenant de réception sont reliées par une conduite de liaison.

2. Dispositif selon la revendication 1, **caractérisé en ce que** le faisceau de membranes de fibres creuses est conçu en forme de U.

3. Dispositif selon la revendication 1 ou 2, **caractérisé en ce que** le module de filtration présente en outre une sortie de rétentat pour l'évacuation du rétentat.

4. Dispositif selon l'une ou plusieurs des revendications 1 à 3, **caractérisé en ce qu'**il comprend au moins deux contenants de réception pour le sérum, qui sont disposés parallèles l'un à l'autre, dans lequel les ouvertures d'entrée des contenants de réception sont reliés à la sortie de perméat du module de filtration respectivement par une conduite de liaison.

5. Dispositif selon l'une ou plusieurs des revendications 1 à 4, **caractérisé en ce qu'**il comprend au moins deux contenants de réception pour le sérum, qui sont disposés en série l'un par rapport à l'autre, dans lequel les contenants de réception sont en communication fluidique l'un avec l'autre et dans lequel l'ouverture d'entrée du premier contenant de réception est reliée à la sortie de perméat du module de filtration par une conduite de liaison.

6. Procédé pour recueillir du sérum acellulaire et stérile à partir de sang total, comprenant les étapes de :

- fourniture du sang total dans une poche à sang,
- coagulation du sang total dans la poche à sang en une composante solide contenant les composants cellulaires du sang et en un liquide contenant du sérum,

- évacuation du liquide contenant le sérum par le biais d'une sortie de la poche à sang, dans lequel la poche à sang présente, dans la zone de la sortie, un dispositif d'arrêt, qui est approprié à retenir au moins une grande partie de la composante liquide,

- introduction du liquide par le biais d'un dispositif d'entrée destiné à introduire le liquide dans un espace de rétentat d'un module de filtration,

- filtration du liquide au moyen d'une membrane semi-perméable disposée dans le module de filtration et appropriée à retenir les composantes solides contenues dans le liquide avec un écoulement transmembranaire dans la plage de 150 000 à 300 000 dm$^3$/(m$^2$·h·MPa) (15 000 à 30 000 L/(m$^2$·h·bar)) ainsi qu'un pore nominal de 0,2 $\mu$m, moyennant quoi un sérum acellulaire et stérile est obtenu en tant que perméat et un rétentat est obtenu, dans lequel subsistent les composantes solides du liquide,

- évacuation du sérum acellulaire par le biais d'une sortie de perméat du module de filtration et

- introduction du sérum acellulaire dans au moins un contenant de réception par le biais d'une ouverture d'entrée dudit au moins un contenant de réception, dans lequel la sortie de perméat et l'ouverture d'entrée sont reliées par une conduite de liaison.

7.  Procédé pour recueillir du sérum acellulaire à partir de sang total selon la revendication 6, **caractérisé en ce que** l'écoulement, hors de la poche à sang dans le module de filtration et, en outre, hors du module de filtration dans ledit au moins un contenant de réception, s'effectue uniquement sous l'influence de la force de gravité.

8.  Procédé pour recueillir du sérum acellulaire à partir de sang total selon la revendication 6 ou 7, **caractérisé en ce qu'**un dispositif selon une ou plusieurs des revendications 1 à 5 est utilisé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2174676 A1
- DE 102009022793 A1
- WO 2014207140 A1
- DE 3617724 A
- WO 2010136535 A

**In der Beschreibung aufgeführte Nicht-Patentliteratur**

- HIMA-Vorschrift. Health Industry Manufacturers Association, 1982, vol. 4